(19) Europäisches Patentamt / European Patent Office / Office européen des brevets

(11) **EP 1 014 176 B1**

(12) **EUROPEAN PATENT SPECIFICATION**

(45) Date of publication and mention
of the grant of the patent:
**29.04.2009  Bulletin 2009/18**

(51) Int Cl.:
*G03C 1/005* (2006.01)     *G03C 1/047* (2006.01)
*C07K 14/78* (2006.01)

(21) Application number: **99204382.8**

(22) Date of filing: **17.12.1999**

(54) **Silver halide emulsions containing recombinant gelatin-like proteins**

Silberhalogenidemulsionen, die rekombinante gelatineartige Proteine enthalten

Emulsions à l'halogénure d'argent contenant des protéines recombinantes semblables à la gelatine

(84) Designated Contracting States:
**DE FR GB NL**

(30) Priority: **23.12.1998  US 219849**

(43) Date of publication of application:
**28.06.2000  Bulletin 2000/26**

(73) Proprietor: **FUJIFILM Manufacturing Europe B.V.**
**5047 TK Tilburg (NL)**

(72) Inventors:
 • **De Wolf, Anton**
  **3981 BR Bunnik (NL)**
 • **Werten, Marc Willem Theodoor**
  **6702 AH Wageningen (NL)**
 • **Wisselink, Hendrik Wouter**
  **6543 TD Nijmegen (NL)**

 • **Jansen-van den Bosch, Tanja Jacoba**
  **6716 NM Ede (NL)**
 • **Toda, Yuzo**
  **5051KZ Goirle (NL)**
 • **van Heerde, George Valentino**
  **4907 XE Oosterhout (NL)**
 • **Bouwstra, Jan Bastiaan**
  **3721 AJ Bilthoven (NL)**

(74) Representative: **van Westenbrugge, Andries et al**
 **Nederlandsch Octrooibureau**
 **Postbus 29720**
 **2502 LS Den Haag (NL)**

(56) References cited:
 **EP-A- 0 926 543     US-A- 5 580 712**
 **US-A- 5 670 616     US-A- 5 710 252**

**Description**

FIELD OF THE INVENTION

The subject invention is directed at tabular silver halide emulsions comprising non-natural gelatin-like proteins for use in improved photographic products.

BACKGROUND OF THE INVENTION

The process of manufacturing photographic paper or triacetate cellulose film consists of coating several layers on top of either a laminated paper or a transparent polymer support. These layers comprise emulsion layers which can contain the photosensitive silver halide crystals as an essential component, and intermediate layers which do not contain these photosensitive components.

There are several stages at which gelatin is used in the process of film-making. The function of the gelatin is different in each stage and thus the required characteristics for each stage are different. Gelatin in numerous forms has been used in photographic manufacturing processes as a peptizer. A peptizer is introduced during the precipitation of the silver halide grains to avoid uncontrolled coalescence. It is well known that the tabular grains with high aspect ratio have several photographic advantages like increased sharpness, improved speed granularity relationships, more rapid developability and higher silver covering power (Research Disclosure Vol. 225 Jan. 1983, Item 22534; EP-A-0.610.796). It has also been desired to produce tabular grains not only with high aspect ratio but also with a narrow grain size distribution otherwise expressed as a desire for mono or homodispersity.

Gelatin used in commercial processes has commonly been derived from animal sources such as animal bone and hide. Disadvantages of this material are the presence of impurities and the fact that the nature of the composition is not clearly defined and thus not reproducible. It is unclear what components are present and in which amounts. In addition it is also unclear which components actually are required for optimal activity. The reproducibility of the photographic manufacturing process is questionable due to the lack of consistency of the gelatin composition used at various stages of the photographic manufacturing process.

US patent US 5,580,712 (Eastman-Kodak) is concerned with modified collagen-like polypeptides and application thereof for photographic purposes and describes that collagen-like peptizers with silver binding strengths below 50 mV can lead to a high degree of thin tabular grain. The document illustrates this for a number of synthetically produced polypeptides with a length of 25 amino acids. The document also mentions one polypeptide with a collagen-like structure was produced using recombinant technology. The recombinant polypeptide is a synthetic polypeptide of block copolymer structure consisting of merely 4 different amino acids.

The problem of the prior art to be solved by the present invention is to provide photographic emulsions at acceptable production costs which emulsions contain collagen-like proteins with homogeneous, reproducible properties allowing an efficient use as peptizer in the emulsions. Prior art methods for producing specific sequences had very low expression rates. Using 20 litres of culture, merely 600 mg of product could be isolated, dissuading the skilled person to use recombinant gelatin-like synthetic material for large-scale photographic applications. This low expression is probably due to the high repetitivity of the sequence to be transcribed and translated and/or to the presence of protease. In particular due to the open structure of non-helical collagan, expression product is likely to be susceptible to protease attack.

In EP 0 926 543 A1, published after the present priority date, applicant has disclosed the production of collagen similar to that of collagen derived from animal sources with high, economically feasible expression rates, without being susceptible to protease attack, and the use of such recombinantly produced material in the preparation of photographic silver halide emulsion with improved properties.

DESCRIPTION OF THE INVENTION

The subject invention is directed at tabular silver halide emulsions comprising non-natural gelatin-like proteins. Such proteins can be produced by recombinant DNA technology. They are particularly useful in photographic applications, more specifically in the production of tabular silver halide emulsions. The invention enables production of large amounts of substantially pure collagen-like without requiring expensive media, expression hosts or non secreting expression hosts. In addition it has now become possible to produce collagen selected and/or adapted for optimal use in each particular stage of the production process of the photographic product where gelatin has to date been applied. Moreover, breakdown due to protease attack in the fermentation process could surprisingly be prevented. Also an improves silver halide emulsion production process is now possible leading to a reduction in production costs. The non-natural collagen-like proteins used in the invention are defined in the claims. They can be homosdisperse or a mixture of homodisperse fractions of protein material. They have a non-helical structure because the proline residues are not hydroxylated and thus do not result in collagen crosslinks.

A non-natural gelatin-like polypeptide or protein is understood according to the invention as a polypeptide having a degree of homology of at least 40% with polypeptide sequences of the same length which are part of natural collagen, but being different form natural sequences. Preferably, the amino acid sequence exhibits more than 50% homology with a native collagen amino acid sequence , more preferably at least 60%. The difference may reside in mutations of native sequences such as insertions, deletions and substitutions vis a vis the native sequence. Besides, use can be made of synthetic DNA sequences with a the appropriate degree of homology with native DNA sequences. The mutations should always result in an amino acid sequence with more than 4 different amino acids, two of them being Gly and Pro. Preferably more than 8, even more than 9 different amino acids should be present. The majority of triads of amino acids (at least 80%) should have the sequence GXY, wherein G is glycine and X and Y are any amino acid, but an occasional deviating triad such as AXY (A = alanine) does not alter the required properties. The GXY motif should not be in the form of a block copolymer of short-chain (e.g. GXY) repetitive units and should not comprise non-GXY spacer sequences between a number of GXY motifs. A substantial number of triads (say, more than half of the total sequence) should have the sequence GXP or GPY (P = proline). Preferably cysteine is avoided.

Proline is not hydroxylated, resulting in non-gelling polypeptides that can be used at low temperature crystallisation without gelling. Such behaviour was not noted before. The low temperature crystallisation process has advantages for the so-called twinning process, resulting in higher surface area of tabular crystals versus non-tabular crystals.

The different stages of film making preferably require collagen with specific characteristics for that stage. In the construction of the non-natural collagen-like proteins according to the invention the flexibility is offered to design tailor made proteins. It may be noted that the proteins according to the invention can also be partly or wholly produced by methods other than DNA expression, e.g. by chemical protein synthesis.

The proteins of the invention do not contain cysteine or another mercapto amino acid, nor do they contain a combination of methionine and arginine in 1-4 position (Met-Xay-Xaz-Arg), as such a sequence is sensitive to enzymatic proteolysis.

The method of production of collagen-like proteins by recombinant technology is described in EP 0 926 543.

The invention in particular pertains to tabular silver halide emulsion nucleated in the presence of at least one peptizer in the form of a protein described above. An emulsion according to the invention wherein the peptizer is present in substantially pure form means that the peptizer is substantially free of nucleic acid, polysaccharides and other protein. The examples illustrate that this is indeed feasible. The presence of sugars and nucleic acid in even trace amounts could have some effects on crystal formation and it was indeed to be questioned whether sufficiently pure recombinant material for the specific photographic application could be produced.

The peptizer can be made by recombinant means. It can also be made de novo from a synthetic nucleic acid sequence. The peptizer protein is a single strain strain, i.e is not crosslinked, and has no helix structure. Various ways of ensuring absence of helix structure are available. For instance ensure the peptizer is free of hydroxyproline and/or free of procollagen and telopeptides. Preferably for photographic applications the peptizer should be free of cysteine. A silver halide emulsion according to the invention, wherein the peptizer is not deaminated is an interesting further embodiment of the invention as is a peptizer with an isoelectric point of 7-10.

We discovered, contradictory to the teaching of US 5,580,712, that an emulsion according to the invention produced with a peptizer with a binding strength for silver higher than 50 mV, can have a high level of tabular grain formation. A suitable peptizer will preferably have a binding strength for silver below 100 mV. Contrary to the prior art teaching the peptizer can have a binding strength for silver between 50-100 mV providing an emulsion with excellent tabular grain percentage.

The silver halide emulsion resulting from application of such collagen like material will suitably exhibit more than 80% tabular grains, preferably more than 90%. Most preferred is a tabular grain percentage higher than 95%. The grains will exhibit an average aspect ratio higher than 3 when determined using the double jet method under the reaction conditions described in the example. Note these reaction conditions are not the optimised reaction conditions used in actual photographic emulsion processes for obtaining the highest aspect ratios but merely provide an indication of whether the material is suitable to achieve high aspect ratios when such optimised conditions are applied. The compounds according to the invention upon application of optimised conditions currently used on normal collagen e.g. using an additional ripening process are expected to exhibit much higher aspect ratios. The applied test is merely a quick indicator of high aspect ratio forming capacity and the person skilled in the art will realise what measures can be taken to enhance the result further.

An emulsion according to the invention offers the advantage over conventional collagen comprising silver halide emulsions that the peptizer is of a homodisperse nature. The crystallisation process by virtue of this fact is also more homogeneous with all the advantages mentioned above. It is possible to add at various stages of the nucleation and growth of the silver halide crystals a further homogenous peptizer also clearly defined and substantially pure thus combining price effectiveness and controlling crystallisation properties in a regulated manner previously not possible with a collagen like peptizer. The nucleation of the silver halide can be performed at relatively low temperatures, i.e. below 45°V, in particular below 45°C. Either of the nucleation peptizer or the growth peptizer can be a recombinant polypeptide according to the invention, or both.

After preparing a silver halide emulsion according to the invention the silver halide emulsion can undergo the standard procedures for preparing a photographic element. The emulsion can be applied in a manner known per se for achieving a silver halide emulsion layer on photographic material wherein the silver halide crystals of said layer have an aspect ratio of 5 or more. Said photographic element is suitably a material sensitive to light, laser or X-ray radiation, said element being selected from black and white reversal film, black and white negative film, colour negative film, colour reversal film, film in which the sensitive photographic components are digitally scanned, black and white reversal paper, black and white paper, colour paper, reversal colour paper, paper in which the sensitive photographic components are sensitized by laser radiation out of a digital database. A photographic element obtained according to such a process is also covered by the invention as well as an element using the direct positive process with internal sensitised silver halide emulsion and elements using heat development.

Another aspect of the invention lies in a process of producing recombinant collagen like polypeptide comprising expression of a collagen like polypeptide encoding nucleic acid sequence by a microorganism to a degree exceeding 0.95 grammes/liter, or even exceeding 2 g/l, said recombinant collagen being free of helix structure. The process can suitably be carried out with a fungal cell or a yeast cell. Suitably the host cell is selected from the group consisting of high expression host cells like *Hansenula, Trichoderma, Aspergillus, Penicillium, Neurospora* and *Pichia*. Fungal and yeast cells are preferred to bacteria as they are less susceptible to improper expression of repetitive sequences. Most preferably the host will not have a high level of proteases that attack the collagen structure expressed. In this respect *Pichia* offers an example of a very suitable expression system. Preferably the micro-organism is free of active post-translational processing mechanism for processing collagen like sequences to fibrils thereby ensuring absence of helix structure in the expression product. Also such a process can occur when the micro-organism is free of active post-translational processing mechanism for processing collagen like sequences to triple helices and/or when the nucleic acid sequence to be expressed is free of procollagen and telopeptide encoding sequences. The host to be used does not require the presence of a gene for expression of prolyl-4-hydroxylase the enzyme required for collagen triple helix assembly contrary to previous suggestions in the art concerning collagen production. The selection of a suitable host cell from known industrial enzyme producing fungal host cells specifically yeast cells on the basis of the required parameters described herein rendering the host cell suitable for expression of recombinant collagen according to the invention suitable for photographic applications in combination with knowledge regarding the host cells and the sequence to be expressed will be possible by a person skilled in the art.

To ensure production of a non cleaved sequence a process according to the invention for producing recombinant collagen like material comprises use of a nucleic acid sequence encoding recombinant collagen amino acid sequence substantially free of protease cleavage sites of protease active in the expression host cell. In the case of *Pichia pastoris* for example and possibly also for other host cells a nucleic acid sequence encoding collagen of which the corresponding amino acid sequence is free of [Leu/Ile/Val/Met]-Xaa-Yaa-Arg wherein Xaa and Yaa correspond to Gly and Pro or other amino acids and at least one of the amino acids between the brackets is amended could be preferred. A preferred process according to the invention comprises use of the microorganism *Pichia pastoris* as expression host.

The process suitably provides expression leading to peptide harvest exceeding 3 grammes /liter. The process can suitably be carried out with any of the recombinant collagen like peptizers defined above for the emulsion according to the invention. As is apparent from the examples under the circumstances described therein multicopy transformants provide more than 14 grams of gelatin per liter of clarified broth at a biomass wet weight of 435 grams per liter. Most suitably the product resulting from microbial expression is isolated and purified until it is substantially free of other protein, polysaccharides and nucleic acid. As is apparent from the examples numerous methods are available to the person skilled in the art to achieve this. The process according to the invention can provide the expression product isolated and purified to at least the following degree: content nucleic acid less than 100 ppm, content polysaccharides less than 5%, content other protein less than in commercial products. More preferably the DNA content of less than 1 ppm, RNA content less than 10 ppm even less than 5 ppm and polysaccharide content less than 0.5% or even less than 0.05% can be achieved.

In a preferred embodiment of the invention the gelatin-like material comprises no cysteine residues. The presence of cysteine in photographic product will disturb the product manufacturing process. It is thus preferred that cysteine is present in as small an amount as possible. Suitably photographic applications will employ material comprising less than 0,1% cysteine.

In particular for the optimal silver halide emulsion homogeneity of the collagen material is of the utmost importance. It is not merely a question of absence of impurities that provides an improvement it is the possibility of providing molecules of exactly the same composition and length allowing good control of the extremely sensitive process of crystallisation and also enabling uniform crystal growth. For this reason recombinant collagen like material will be valuable for this part of the photographic manufacturing process. In addition the absence of fibril formation and even of triple helices is required for this particular application in the photographic manufacturing process an aspect that until now had been completely overlooked. The insight in the relevance of the number of reducing groups in the photographic material is also of great importance. This is not the rigid low amount suggested in the prior art required for tabular grain formation. The invention will further be illustrated by the examples.

Gelatins, collagen or collagen fragments expressed as recombinant, heterologous protein in expression host organisms like yeast, fungi, bacteria for photographic applications by recombinant-DNA techniques has several advantages. (i) In contrast to for example traditional gelatins, recombinant molecules can be produced as rigorously non cross-linked. (ii) The molecular composition is precisely defined. (iii) The molecules produced are of a single type (or a well-defined mixture of only a few molecules), with minor or negligible contamination from other proteinaceous or non-proteinaceous molecules. The molecular weight distribution is very narrow and mono-disperse (single-component gelatins) or oligo-disperse. (iv) The product can be manufactured in a highly reproducible way, i.e. with constant quality. Especially yeast are well-suited production organisms for such polypeptides with a highly repetitive, glycine- and proline-rich sequence.

Several strong and tightly-regulated inducible promoters are available for yeast systems, allowing a highly efficient expression and minimising possible negative effects on the viability and growth of the host cells. As one of several well-suited systems that are available, we have chosen for secreted production by the methylotrophic yeast *Pichia pastoris*. After transformation of the host, the integrative is incorporated into the yeast's genome, resulting in genetical stability of the transformants (loss of plasmids is then of no importance). It is possible to generate transformants (with the heterologous target gene under the control of e.g. the alcohol oxidase (AOX) promotor), in which the recombinant gene is either incorporated into the *HIS4* locus or the *AOX1* locus. In the latter case, depending on the type of integration, the *AOX1*-gene is disrupted, leading to slow utilisation of (and slow growth on) methanol (Mut^S phenotype). If the functional AOX1 gene is still present, the phenotype is Mut+. Although both phenotypes can be used, we generally preferred fast growth and thus, our protocols were mainly directed at the generation and selection of Mut+ transformants. It is self-evident that yeast or fungal expression systems other than the *P. pastoris* expression system could in principle be used equally well for the efficient production of recombinant gelatins, depending on the exact type and quality of molecule to be produced, on the production scale envisaged, and on the production costs and applicable market prices. The *Pichia* system was used as a fast and efficient system for pilot production and relatively easy product recovery.

## EXAMPLES

## MATERIALS, METHODS AND ANALYSES

### General molecular-biological techniques

Cloning procedures were carried out essentially according to Maniatis *et al.* [3]. DNA was isolated using Wizard Plus SV miniprep, or Qiagen midiprep systems. DNA was isolated from agarose gels using the QIAquick Gel Extraction Kit (Qiagen). All enzymes used were from Pharmacia unless otherwise stated and were used according to the manufacturer's recommendations. Transformation of *E. coli* was performed by standard electroporation using the BioRad GenePulser. All procedures involving the handling and transformation of *Pichia pastoris* and the expression of proteins in this host organism were essentially carried out according to the manual of the *Pichia* Expression Kit (from Invitrogen) [4].

## EXAMPLE 1

### Construction and expression of synthetic gene.

A synthetic gene, encoding a polar gelatin (P monomer) was constructed by overlap extension PCR. The theoretical molecular weight and isoelectric point are 9.1 kD and 4.9, respectively. The gene was designed to have the codon usage of Pichia pastoris highly expressed genes (Sreekrishna, K. and Kropp, K.E. (1996) Pichia pastoris, Wolf, K. (Ed), Nonconventional yeasts in biotechnology. A handbook, Springer-Verlag , pp. 6/203-6/253). Two separate PCR reactions were performed, using the following oligonucleotides:

1 pmol OVL-PA-FW, 1 pmol OVL-PA-RV, 50 pmols HLP-PA-FW and 50 pmols HLP-PA-RV.
1 pmol OVL-PB-FW, 1 pmol OVL-PB-RV, 50 pmols HLP-PB-FW and 50 pmols HLP-PB-RV.

Oligonucleotide sequences were as follows:

HLP-PA-FW: 5'-GCGCTCGAGAAAAGAGAGGCTGAAGC-3'
OVL-PA-FW: 5'-GCGCTCGAGA AAAGAGAGGC TGAAGCTGGT CCACCCGGTG

5

AGCCAGGTAA CCCAGGATCT CCTGGTAACC AAGGACAGCC CGGTAACAAG GGTTCTCCAG GTAATCCA-3'

OVL-PA-RV: 5'-TGAGAACCTT GTGGACCGTT GGAACCTGGC TCACCAGGTT

GTCCGTTCTG ACCAGGTTGA CCAGGTTGAC CTTCGTTTCC TGGTTGACCT GGATTACCTG GAGAACCCTT-3'

HLP-PA-RV: 5'-TGAGAACCTT GTGGACCGTT GGAA-3'
HLP-PB-FW: 5'-TTCCAACGGT CCACAAGGTT CTCA-3'

OVL-PB-FW: 5'-TTCCAACGGT CCACAAGGTT CTCAGGGTAA CCCTGGAAAG AATGGTCAAC CTGGATCCCC AGGTTCACAA GGCTCTCCAG GTAACCAAGG TTCCCCTGGT CAGCCAGGTA ACCCT-3'

OVL-PB-RV:

5'-GCGTCTGCAG TACGAATTCT ATTAGCCACC GGCTGGACCC TGGTTTCCTG GTTTACCTTG TTCACCTGGT TGACCAGGGT TACCTGGCTG ACCAGGGGAA CCTTGGTT-3'

HLP-PB-RV: 5'-GCGTCTGCAG TACGAATTCT ATTAGC-3'

The 50 μl PCR reactions contained 0.2mM dNTP's (Pharmacia), 1x Pwo buffer (Eurogentec) and 1.25 U Pwo polymerase (Eurogentec). Reaction 1 involved 18 cycles consisting of 15 seconds at 94 °C and 15 seconds at 72 °C. Reaction 2 involved a touchdown PCR, whereby each cycle consisted of 15 seconds at 94°C, 15 seconds at the annealing temperature and 15 seconds at 72 °C. The annealing temperature was lowered from 72 °C to 68 °C in the first 5 cycles, after which 20 additional cycles at an annealing temperature of 67 °C were performed.

The PCR products were isolated from agarose gel. 0.3 pmols of each fragment and 50 pmols of the outer primers HLP-PA-FW and HLP-PB-RV were subjected to overlap extension PCR. 25 cycles consisting of 15 seconds at 94 °C, 15 seconds at 67°C and 15 seconds at 72 °C were performed. The resulting 0.3 kb PCR fragment was digested with XhoI/EcoRI and cloned in cloning vector pMTL23. The sequence of the gene was verified by automated DNA sequencing.

In order to create a P tetramer (P4; theoretical molecular weight 36.8 kD), the fragment was released by digesting the vector with DraIII/Van91I. In a separate reaction the vector was digested with Van91I and dephosphorylated. The DraIII/Van91I fragment was then inserted into this Van91I digested vector. This yielded a vector containing a P dimer. This dimer was released by digestion with DraIII/Van91I and reinserted into the Van91I site of the dimer bearing vector, yielding the P tetramer (P4). The P and P4 fragments were then cloned into the XhoI/EcoRI sites of vector pPIC9. The encoded amino acid sequence of the mature (processed) P monomer and tetramer are as follows:

Monomer (P):

```
-3      GPP
 1      GEP GNP GSP GNQ GQP GNK GSP GNP GQP GNE GQP
34      GQP GQN GQP GEP GSN GPQ GSQ GNP GKN GQP GSP
67      GSQ GSP GNQ GSP GQP GNP GQP GEQ GKP GNQ GPA
100     GG
```

Tetramer (P4):

```
  1     GPPGEPGNPG SPGNQGQPGN KGSPGNPGQP GNEGQPGQPG QNGQPGEPGS
 51     NGPQGSQGNP GKNGQPGSPG SQGSPGNQGS PGQPGNPGQP GEQGKPGNQG
101     PAGEPGNPGS PGNQGQPGNK GSPGNPGQPG NEGQPGQPGQ NGQPGEPGSN
151     GPQGSQGNPG KNGQPGSPGS QGSPGNQGSP GQPGNPGQPG EQGKPGNQGP
201     AGEPGNPGSP GNQGQPGNKG SPGNPGQPGN EGQPGQPGQN GQPGEPGSNG
251     PQGSQGNPGK NGQPGSPGSQ GSPGNQGSPG QPGNPGQPGE QGKPGNQGPA
301     GEPGNPGSPG NQGQPGNKGS PGNPGQPGNE GQPGQPGQNG QPGEPGSNGP
351     QGSQGNPGKN GQPGSPGSQG SPGNQGSPGQ PGNPGQPGEQ GKPGNQGPAG
401     G
```

The vectors were linearized by digestion with SalI and were used to transform *Pichia pastoris* strain GS115. Fermentations were performed as described for COL1A1 (i.e. growth at pH 3 and in the presence of casamino acids). Culture supernatants were analysed by SDS-PAGE and revealed protein bands having the expected N-terminal amino acid sequences. The yield was calculated to be 1 gram/liter fermentation medium. The products could be purified by acetone fractionation as described for the native gelatins (i.e. removal of endogenous proteins at 40% acetone and precipitation of gelatin at 80% acetone).

*Expression / production of gelatin in a fermentor*

Fed-batch fermentations were performed according to the *Pichia* fermentation process guidelines of Invitrogen. Cells were grown in a 1-liter fermentor (Applikon) in the initial experimental stages to optimize protein production. Thereafter cells were grown in a 20-liter or a 140-liter fermentor (Biobench 20, Bio-pilot 140, Applikon) for pilot scale production of collagen. Working volumes were 1-liter, 15-liter and 100-liter, respectively. AD1020 controllers (Applikon) were used to monitor and control the fermentation parameters. The program BioXpert (Applikon) was used for data storage. Dissolved oxygen levels were monitored in the fermentor using an oxygen electrode (Ingold for 1-liter fermentations, Mettler Toledo for larger scale fermentations). Agitation (500 - 1000 rpm) and aeration (1 - 2 vvm, i.e. 1-2 $LL^{-1}min^{-1}$) were manually adjusted to keep the dissolved oxygen concentration above 20%. pH was measured by a pH electrode (Broadly James cooperation) and automatically kept at pH 3.0 or pH 5.0 by addition of ammonium hydroxide (25%), which also served as nitrogen source for growth of the microorganisms. An anti foam-electrode was used to prevent excessive foaming. When necessary, the anti foam Structol J673 (Schill and Seilacher, Hamburg, Germany) was used. Growth of the microorganisms was monitored by determination of the cell dry weight. A calibration curve was made by which cell wet weight could be converted into cell dry weight. Cell wet weight was determined after centrifugation of 2 ml-samples for 5 min at 15.000 rpm and removing the supernatant. Cell dry weight was determined after addition of 200 μl of cells to a pre-dried filter (0.45 μm membrane, Schleicher & Schüll, Dassel, Germany), washing with 25 ml of deionized water and drying e.g. in a microwave oven for 15 minutes at 1000 W. Cell dry weight was approximately a factor 3 lower than cell wet weight. Precultures were started from colonies on a MGY plate, in flasks containing a total of 10% of the initial fermentation volume of MGY. The volume of the medium was ≤20% of the total flask volume. Cells were grown at 30°C at 200 rpm in a rotary shaker (Gallenkamp) for 24-60 hours.

*Fermentation medium*

The fermentation basal salts medium in the fermentor contained per liter: 26.7 ml of phosphoric acid (85%), 0.93 g calcium sulfate, 18.2 g potassium sulfate, 14.9 g magnesium sulfate.7$H_2$O, 4.13 g potassium hydroxide and 40.0 g glycerol. An amount of 4.3 ml of $PTM_1$ trace salts was added per liter of fermentation basal salts medium. $PTM_1$ trace salts contained per liter: 4.5 g cupric chloride.2$H_2$O, 0.09 g potassium iodide, 3.5 g manganese chloride.4$H_2$O, 0.2 g sodium molybdate.2$H_2$O, 0.02 g boric acid, 1.08 g cobalt sulfate.7$H_2$O, 42.3 g zinc sulfate.7$H_2$O, 65.0 g ferrous sulfate. 7HO, 0.2 g biotin and 5.0 ml sulfuric acid. Trace salts were filter sterilised (pore size 0.22 $\mu$m, Costar, USA).

At the end of the fermentation, the cells were removed by centrifugation (10.000 rpm, 30 min, 4°C), followed by micro filtration (cut off 0.2 $\mu$m) in the case of the 1 litre fermentation. Cells were removed by micro filtration in the case of the 20 or 100 litre fermentation.

In the case of 20 L fermentation, the cell broth was applied to a micro filtration module containing a poly ether sulphone membrane with 0.20 $\mu$m pore size (type MF 02 M1 from X-Flow, fitted in a RX 300 filtration module from X-Flow). In the case of the 100-liter fermentation cells were removed by a pilot plant scale cross flow micro filtration unit containing a hollow fiber poly ether sulphone membrane with 0.2 $\mu$m pore size (type MF 02 M1, from X-Flow, fitted into a R-10 membrane module). These filtration units are mentioned merely as examples. It will be understood that any suitable micro filtration system could be applied to remove the cells. Optionally, the bulk of cells and debris was removed by centrifugation, and only the supernatant and the medium used to wash the cells was applied to the micro filtration units.

In the case of the 100 Litre fermentation, the cell broth was first applied to a filtration unit fitted with a stack of flat 0.4x0.4 m cellulose Bio 10 or Bio 40 depth filters (USF Seitz Filter Werke, Bad Kreutznach, Germany) with a total filtration surface of 1.9 $m^2$. Thereafter, the permeate was filtered with a spiral wound 0.2 $\mu$m pore size polysulphone dead end micro filtration unit (USF Seitz Filter Werke, Bad Kreutznach, Germany). After micro filtration, the filters were sterilized, the cells were destroyed by steam sterilization or by autoclaving, and the absence of recombinant Pichia cells in the filtrate was verified by plating out samples of filtrate.

*Purification of synthetic gelatins from the cell free fermentation broth*

*Separation of recombinant bipolar gelatines and non-recombinant Pichia proteins or small peptides (optional).*

For separation of recombinant bipolar gelatines and non-recombinant Pichia proteins, cell-free fermentation broth was subjected to differential precipitation (= fractionation) at 40-80 volume-% ethanol or acetone. At 40 volume-% ethanol or acetone, the non-gelatineous proteins (from Pichia) were precipitated, while at 60-80 volume-% ethanol or acetone, gelatine was precipitated, as shown by SDS-PAGE and analysis of the amino acid composition. Small peptides and other low molecular weight contaminants remained in solution at 80 vol.-% ethanol or acetone. Ethanol or acetone was cooled for 2-4 hours at -20°C. An amount of 40 vol.-% of ice-cold ethanol or acetone (v/v) was added slowly to the pre-cooled supernatant from the fermentation at 4°C under magnetic stirring. Supernatant was stirred overnight at 4°C. Precipitated proteins and particles were removed by centrifugation (4°C, 10.000 rpm, 30 min). The pellet was resuspended in 40 vol.-% ice-cold ethanol or acetone and again centrifuged. Both 40 vol.-% acetone supernatant fractions were pooled. Thereafter the supernatant was brought to 60-80 vol.-% ethanol, or acetone (v/v) and stirred overnight. Precipitated proteins were collected by centrifugation. The pellet was dissolved in an appropriate amount of water. In addition to ultra filtration or evaporation of water, precipitation of gelatine at 80 % (70-90 %) ethanol or acetone can also be used to concentrate the protein.

*Purification of recombinant gelatine from fermentation broth by anion exchange chromatography.*

At laboratory (mg to g) scale, the recombinant gelatine was optionally captured and purified from the fermentation broth by anion exchange chromatography (e. g. using Q Sepharose HP or XL from Amersham Pharmacia Biotech, Uppsala, Sweden), preferably in 20 mM phosphate, carbonate or borate buffer at pH 6 to 8 and using a NaCl gradient or step gradient for elution.

*Purification of recombinant gelatine from cell free fermentation broth by ammonium sulphate precipitation.*

The recombinant gelatine was purified from non-recombinant proteins and peptides, polysaccharides, nucleic acids and other contaminating molecules by selective precipitation of the gelatine at 60 % saturation of ammonium sulphate. Ammonium sulphate was slowly added to 60% saturation at 4°C. After 60 min stirring the sample was centrifuged (30 min, 4°C, 10.000 rpm). The pellet was resuspended in 60% ammonium sulphate and again centrifuged. If more than 1 % (w/w) polysaccharides or sugars remained present, the ammonium sulphate precipitation procedure described above was repeated after complete redissolving of the gelatine in the absence of ammonium sulphate. Alternatively, the ammonium sulphate precipitate was collected on a suitable depth filter (e.g. Bio 10, Bio 40, or AKS5 from USF Seitz Filter Werke, Bad Kreuznach, Germany) and washed free from contaminating components by flushing the filter and filter cake with 60-70 % ammonium sulphate.

Finally, the purified gelatine pellet or filter cake was dissolved in de-ionised water.

At laboratory scale, milligram to gram quantities of purified recombinant gelatine were desalted by dialysis against deionised water, which was refreshed every 4 hours. Dialysis membranes of regenerated cellulose (Spectra Por®, from Spektrum) were used with a molecular weight cut-off of 8 kD. The dialysis was stopped after 2-7 days when the electrical conductivity of the sample was judged to be sufficiently low.

At pilot scale (i.e. gelatine quantities of more than 10 to 100 gram), the purified recombinant gelatine was desalted by ultra filtration / dia filtration, using poly ether sulphone membranes with a molecular cut-off of 4 (1 to 10) kD. The ultra filtration dia filtration was stopped when the electrical conductivity of the gelatine solution was sufficiently low. This typically occurred after 10 to 30 cycles of dilution (2x) and concentration (2x).

Optionally, an additional, final desalting step was carried out by adding a slight excess of mixed-bed ion exchange resin beads (e.g. Amberlite MB-3 from Merck, Darmstadt, Germany) and incubating for 30 min. to maximally 1 hour.

Conductivity was measured with a digital conductivity meter (Radiometer), calibrated with 1 mM and 10 mM KCl solutions (140 and 1400 $\mu$S.cm$^{-1}$, respectively). After desalting by dialysis or diafiltration, the final electrical conductivity was typically 5 to 15 $\mu$S.cm$^{-1}$ .((gram gelatine).L$^{-1}$)$^{-1}$. After further desalting with mixed bed ion exchange beads, the final electrical conductivity was typically 0.5 to 3 $\mu$S.cm$^{-1}$.((gram gelatine).L$^{-1}$)$^{-1}$.

Where applicable, the product was pre-dried (optional) by precipitation with high concentrations of acetone and evaporation of the acetone.

The purified and desalted product was either freeze-dried or spray-dried.

*Characterisation of the gelatine product*

*Molecular weight distribution and contaminating proteins*

The molecular weight distribution of the recombinant gelatine product and the presence of contaminating proteins were analyzed by denaturing poly acrylamide gel electrophoresis (SDS-PAGE) [6] in a Mini-PROTEAN II system (from Biorad) and Coomassie Brilliant blue staining. Optionally, a higher voltage was applied (400 Volt), in order to speed-up the electrophoresis rate and minimize protein diffusion. For both bipolar gelatine types a single gelatine band was observed. After purification as described above (e.g. micro filtration, ammonium sulphate fractionation and desalting), the product was apparently homogeneous and free from contaminating proteins.

The molecular weight apparent from SDS-PAGE, as calibrated with globular protein molecular weight markers, was too high, in accordance with numerous other observations on gelatines and collagen-like proteins and polypeptides.

However, after gel filtration of the ammonium sulphate-purified gelatine with a Superose-12 column (Amersham Pharmacia Biotech, Uppsala, Sweden), a single gelatine peak eluted at the correct theoretical molecular weight, with reference to a set of 6 distinct fragments of recombinant mouse type I collagen, having known (i.e. theoretical and experimentally verified) molecular weights of 8, 12, 16, 28, 42 an 54 kDa. The gelatine samples were eluted from the Superose-12 column with 100 mM NaCl and a flow of 0.2 mL/min. This showed that the recombinant gelatine product was correctly expressed and was not degraded to any considerable extent. The correctness of molecular weight was confirmed by mass spectrometry.

*Confirmation of N-terminal amino acid sequence*

After SDS-PAGE as described above, the proteins in the gel were blotted onto an Immobilon P$^{SQ}$ membrane (from Millipore) by applying 100 V for one hour in a Mini Trans-Blot Cell (Biorad). Transfer buffer was 2.2 g CAPS per litre of 10 % methanol, pH 11. Blots were stained with Coomassie Brilliant Blue and selected bands were cut out. N-terminal protein sequencing was performed by Edman degradation. It appeared that the N-terminal sequence of both recombinant gelatine products was correct.

*Confirmation of purity and amino acid composition*

The amino acid composition of the purified gelatine product was determined after complete HCl-mediated hydrolysis of the peptide bonds at elevated temperature, followed by derivatisation of the amino acids with a fluorophore, and HPLC.

The percentage Gly expected from pure gelatin is 33%. This offers a means of estimating the purity of produced recombinant gelatines. In order to correct for the percentage of Gly in endogenously secreted proteins of *P. pastoris*, amino acid composition analysis was performed on fermentation supernatant of a Mut$^{+}$ transformant of pPIC9. The percentage Gly found was 9%. The purity of a sample can now be estimated by the formula:

$$(\%Gly-9)/(33-9) = (\%Gly-9)/24.$$

*Determination of contamination with polysaccharides, sugars and nucleic acids*

After dissolution of samples in MilliQ water, the following assays were performed. The sugar content was determined by a phenol-based assay. 200 $\mu$L samples were mixed with 200 $\mu$l 5 % (w/w) phenol. After thorough mixing using a Vortex mixer, 1 mL of concentrated sulphuric acid was added. After mixing, the samples were incubated for 10 min at room temperature and, subsequently, for 20 min. at 30 °C. After cooling, the light absorption of the samples at 485 nm was determined. Analytical grade mannose was used to prepare the calibration curve.

The DNA content was determined by mixing aliquots of diluted SYBR$_\circledR$ Green I nucleic acid 'gel' stain (10.000 x conc. In DMSO) from Molecular Probes with our samples. After thorough spectral analysis, the excitation wavelength was chosen to be 490 nm, and the emission wavelength 523 nm. The calibration was by subsequent addition of known amounts of DNA to this same mixture, as internal standards. Thus, a calibration curve was constructed. Furthermore, it was checked that subsequent addition of DNA-degrading enzyme resulted in complete break down of the fluorescent signal.

A quantitative indication of the RNA plus DNA-content was subsequently obtained by using SYBR$_\circledR$ Green II 'RNA gel stain', instead of SYBR$_\circledR$ Green I. After thorough spectral analysis, the excitation wavelength was chosen to be 490 nm, and the emission wavelength 514 nm. Calibration was by subsequent addition of known amounts of RNA. The resulting value was pronounced to be the 'RNA' content of the sample. In the absence of DNA, it corresponded to the true RNA content. When present, the DNA-associated fluorescence may have biased the RNA values, although a final addition of RNAse was used to discern the DNA- and RNA-derived contributions to the fluorescence.

*Quantification of the recombinant product*

The protein content was determined by the BCA assay from Pierce, using gelatine from Merck (Darmstadt, Germany) as a reference. The gelatine content of the cell-free fermentation broth and various semi-purified gelatine samples was determined by fractionating the samples at 40 and 80 volume-% of ethanol or acetone, as described above, and quantifying the protein content of the 40 % pellet (i.e. non-recombinant Pichia protein), the pellet obtained by enhancing the solvent content of the 40 % supernatant to 80 vol.-% (i.e. precipitated recombinant gelatine), as well as the 80 % supernatant (small peptides and bias from other molecules). Again the BCA assay from Pierce was applied, using gelatine from Merck as a reference. The purified and dried product was in addition quantified by weight determination.

RESULTS:

Gelatine batch produced at laboratory scale (example)

- about 50 gram
- purification: micro filtration, $(NH_4)_2SO_4$, desalting by diafiltration, lyophilisation.
- DNA: 2 ppm (w/w)
- RNA: 10 ppm (w/w)
- total sugars: < 0.05 % (w/w)
- purity calculated from amino acid composition determination >99%
- conductivity: 4.2 $\mu$S.cm$^{-1}$.((gram gelatin).L$^{-1}$)$^{-1}$ corresponding to less than 750 ppm free $(NH_4)_2SO_4$.
- gelatin was single-component according to SDS-PAGE and FPLC.

REFERENCES

[1] Capello, J. & Ferrari, F. (1994) in: Plastics from microbes (Mobley, D. P., ed.) Hanser, Munich, pp. 35-92
[2] Strausberg, R. L. & Link, R. P. (1990) TIBTECH 8: 53-57.
[3] Maniatis T., Fritsch, E. F. & Sambrook, J. (1982) Molecular cloning: A laboratory manual. Cold Spring Harbor Laboratory, Cold Spring Harbor, NY.

[4] Manual of the *Pichia* Expression Kit Version E (Invitrogen, San Diego, CA, USA).
[5]

- Glumoff, V., Mäkelä, J. K. & Vuorio, E (1994) Cloning of cDNA for rat proα1(III) collagen mRNA. Increased expression of type III collagen gene during induction of experimental granulation tissue. Biochim Biophys Acta 1217: 41-48.
- EMBL/GenBank accession number X70369.

[6] Sanger, F., Nicklen, S. & Coulson, A. R. (1977) Proc. Natl. Acad. Sci. USA 74: 5463-5467.
[7] Becker, D. M. & Guarente, L. (1991) High efficiency transformation of yeast by electoporation. Methods in Enzymology, vol. 194: 182-187.
[8] Lee, F. J. S. (1992) Biotechniques 12 (5): 677
[9] Laemmli, U. K. (1970) Cleavage of structural proteins during the assembly of the head of bacteriophage T4. Nature 227: 680-685.
[10] Schmitt, M. E., Brown, T. A. and Trumpower, B. L. (1990) A rapid and simple method for preparation of RNA from Saccharomyces cerevisiae. Nucleic Acids Res. 18 (10): 3091.
[11] Scorer, C. A., Clare, J. J., McCombie, W. R, Romanos, M. A. & Sreekrishna, K. (1994) Rapid Selection using G418 of high copy number transformants of Pichia pastoris for high-level foreign gene expression. Bio/Technology 12: 181-184.
[12] Butkowsky R. J., Noelken, M. E. & Hudson, B. G. (1982) Estimation of the size of colagenous proteins by electrophoresis and gel chromatography. Meth. Enzymol. 82: 410-423.
[13] De Wolf, F. A.& Keller R. C. A (1996) Characterization of the helical structures in gelatin networks and model polypeptides by circular dichroism. Progr. Colloid Polym. Sci. 102: 9-14.

## Example 2

*Preparation of silver bromide crystals with non-natural recombinant gelatins*.

Nucleation: The nucleation is performed applying the same conditions (also pH=5.5) as in the comparative example except that the gelatin in the reaction mixture is replaced by the gelatin sample of the invention.

Ripening: The ripening is done according to the same procedure as is used in the comparative example.

Result: A high % tabular grains of more than 85% with an average aspect ratio of 5:1 is shown in table II.

## Comparative example 1 (control)

*Preparation of silver bromide crystals with conventional regular bone gelatin*.

Nucleation: At a temperature of 35°C in a reaction vessel containing 8.0 g/l regular gelatin (a standard deionised IAG bone gelatin from PB Gelatins, Tessenderlo, Belgium) and 12.7 mM potassium bromide the pH is adjusted to a value of 5.5 by sodium hydroxide or sulphuric acid. By double jet addition aqueous solutions of 1.62 M silver nitrate and 1.64 M potassium bromide are added simultaneously at an equal and constant rate in a period of 2 sec while vigorously stirring by a propeller mixer. After the nucleation reaction as described above the bromide ion concentration is increased to 21 mmol/l by the addition of 3.4 M potassium bromide.

Ripening: Subsequently the content of the vessel is transferred to a ripening vessel, where a second type of gelatin is added in a quantity sufficient to bring its concentration to 14.2 g/l. The temperature is increased gradually to a value of 75°C and the ripening reaction is continued for 30 minutes. At the end of this period the Ostwald ripening rate is stopped by adding a solution of methyl phenyl tetrazole and cooling to room temperature. A sample of the prepared emulsion was analysed by transmission electron microscopy as replica thereof.

Result: a low % of tabular grains is formed (<20%)

## Comparative example 2 (control)

*Preparation of silver bromide crystals with conventional hydrolysed bone gelatin*.

Nucleation: the nucleation is performed applying the same conditions (also pH=5.5) as in the comparative example 1 except that the gelatin in the reaction mixture is replaced a deionised hydrolysed bone gelatin (supplied by Nitta Gelatin in Japan).

Ripening: the ripening is done according the same procedure as is used in the comparative example 1.

Result: a low % of tabular grains is formed (<20%).

**Example 3 (invention)**

*Preparation of silver bromide crystals with non-natural recombinant gelatin.*

Nucleation: the nucleation is performed applying the same conditions (also pH=5.5) as in the comparative example 1 except that the gelatin in the reaction mixture is replaced by the gelatin sample of the invention.
Ripening: the ripening is done according the same procedure as is used in the comparative example 1.
Result: a high % tabular grains that account for more than 80% of the projected surface area as is shown in table II.

**Claims**

1.  A tabular silver halide emulsion wherein the tabular grains account for more than 75% of the total grain projected area said emulsion comprising silver halide grains nucleated in the presence of a nucleation peptizer and thereafter grown in the presence of a growth peptizer, wherein either of the nucleation peptizer or the growth peptizer, or both, is a substantially pure recombinant collagen like material free of helix structure and said peptizer having an amino acid sequence comprising more than 4 different amino acids, wherein said peptizer comprises the amino acid sequence:

    GPP GEP GNP GSP GNQ GQP GNK GSP GNP GQP GNE GQP GQP GQN GQP
    GEP GSN GPQ GSQ GNP GKN GQP GSP GSQ GSP GNQ GSP GQP GNP GQP
    GEQ GKP GNQ GPA GG.

2.  A tabular silver halide emulsion according to claim 1, wherein said peptizer comprises the amino acid sequence:

    GPPGEPGNPGS PGNQGQPGNKGS PGNPGQPGNEGQPGQPGQNGQPGEPGS
    NGPQGSQGNPGKNGQPGS PGSQGS PGNQGS PGQPGNPGQPGEQGKPGNQG
    PAGEPGNPGS PGNQGQPGNKGS PGN PGQPGNEGQPGQPGQNGQPGEPGSN
    GPQGSQGNPGKNGQPGS PGSQGS PGNQGS PGQPGNPGQPGEQGKPGNQGP
    AGEPGNPGS PGNQGQPGNKGS PGNPGQPGNEGQPGQPGQNGQPGEPGSNG
    PQGSQGNPGKNGQPGS PGSQGS PGNQGS PGQPGNPGQPGEQGKPGNQGPA
    GEPGNPGS PGNQGQPGNKGS PGNPGQPGNEGQPGQPGQNGQPGEPGSNGP
    QGSQGNPGKNGQPGS PGSQGS PGNQGS PGQPGNPGQPGEQGKPGNQGPAG
    G.

3.  A protein comprising the amino acid sequence:

    GPP GEP GNP GSP GNQ GQP GNK GSP GNP GQP GNE GQP GQP GQN GQP
    GEP GSN GPQ GSQ GNP GKN GQP GSP GSQ GSP GNQ GSP GQP GNP GQP
    GEQ GKP GNQ GPA GG.

4.  A protein comprising the amino acid sequence:

    GPPGEPGNPGS PGNQGQPGNKGS PGNPGQPGNEGQPGQPGQNGQPGEPGS
    NGPQGSQGNPGKNGQPGS PGSQGS PGNQGS PGQPGNPGQPGEQGKPGNQG

PAGEPGNPGSPGNQGQPGNKGSPGNPGQPGNEGQPGQPGQNGQPGEPGSN

GPQGSQGNPGKNGQPGSPGSQGSPGNQGSPGQPGNPGQPGEQGKPGNQGP

AGEPGNPGSPGNQGQPGNKGSPGNPGQPGNEGQPGQPGQNGQPGEPGSNG

PQGSQGNPGKNGQPGSPGSQGSPGNQGSPGQPGNPGQPGEQGKPGNQGPA

GEPGNPGSPGNQGQPGNKGSPGNPGQPGNEGQPGQPGQNGQPGEPGSNGP

QGSQGNPGKNGQPGSPGSQGSPGNQGSPGQPGNPGQPGEQGKPGNQGPAG

G.

## Patentansprüche

1. Flache Silberhalogenidemulsion, wobei die flachen Körner mehr als 75% der gesamten Kornprojektionsflache ausmachen, und die Emulsion Silberhalogenidkörner enthält, welche sich in Anwesenheit eines kernbildenden Peptisierungsmittels bilden und anschließend in Anwesenheit eines wachstumsunterstützenden Peptisierungsmittels gewachsen sind, wobei entweder das kernbildende oder das wachstumsunterstützende Peptisierungsmittel, oder beide, aus einem im Wesentlichen reinen, rekombinanten, collagenartigen Material ausgebildet sind, welches frei von Helixstrukt ist, und das Peptisierungsmittel eine Aminosäuresequenz aus mehr als 4 verschiedenen Aminosäuren aufweist, wobei das Peptisierungsmittel die Aminosäuresequenzen aufweist:

GPP GEP GNP GSP GNQ GQP GNK GSP GNP GQP GNE GQP GQP GQN GQP
GEP GSN GPQ GSQ GNP GKN GQP GSP GSQ GSP GNQ GSP GQP GNP GQP
GEQ GKP GNQ GPA GG.

2. Emulsion nach Anspruch 1, wobei das besagte Peptisierungsmittel die folgende Aminosäuresequenz aufweist:

GPPGEPGNPGSPGNQGQPGNKGSPGNPGQPGNEGQPGQPGQNGQPGEPGS

NGPQGSQGNPGKNGQPGSPGSQGSPGNQGSPGQPGNPGQPGEQGKPGNQG

PAGEPGNPGSPGNQGQPGNKGSPGNPGQPGNEGQPGQPGQNGQPGEPGSN

GPQGSQGNPGKNGQPGSPGSQGSPGNQGSPGQPGNPGQPGEQGKPGNQGP

AGEPGNPGSPGNQGQPGNKGSPGNPGQPGNEGQPGQPGQNGQPGEPGSNG

PQGSQGNPGKNGQPGSPGSQGSPGNQGSPGQPGNPGQPGEQGKPGNQGPA

GEPGNPGSPGNQGQPGNKGSPGNPGQPGNEGQPGQPGQNGQPGEPGSNGP

QGSQGNPGKNGQPGSPGSQGSPGNQGSPGQPGNPGQPGEQGKPGNQGPAG

G.

**3.** Ein Protein, welches die folgende Aminosäuresequenz aufweist:

GPP GEP GNP GSP GNQ GQP GNK GSP GNP GQP GNE GQP GQP GQN GQP
GEP GSN GPQ GSQ GNP GKN GQP GSP GSQ GSP GNQ GSP GQP GNP GQP
GEQ GKP GNQ GPA GG.

**4.** Ein Protein, welches die folgende Aminosäuresequenz aufweist:

GPPGEPGNPGSPGNQGQPGNKGSPGNPGQPGNEGQPGQPGQNGQPGEPGS
NGPQGSQGNPGKNGQPGSPGSQGSPGNQGSPGQPGNPGQPGEQGKPGNQG
PAGEPGNPGSPGNQGQPGNKGSPGNPGQPGNEGQPGQPGQNGQPGEPGSN
GPQGSQGNPGKNGQPGSPGSQGSPGNQGSPGQPGNPGQPGEQGKPGNQGP
AGEPGNPGSPGNQGQPGNKGSPGNPGQPGNEGQPGQPGQNGQPGEPGSNG
PQGSQGNPGKNGQPGSPGSQGSPGNQGSPGQPGNPGQPGEQGKPGNQGPA
GEPGNPGSPGNQGQPGNKGSPGNPGQPGNEGQPGQPGQNGQPGEPGSNGP
QGSQGNPGKNGQPGSPGSQGSPGNQGSPGQPGNPGQPGEQGKPGNQGPAG
G.

**Revendications**

**1.** Emulsion d'halogénure d'argent tabulaire dans laquelle les grains tabulaires comptent pour plus de 75 % de la zone projetée de grain totale, ladite émulsion comprenant des grains d'halogénure d'argent nucléés en présence d'un peptisant de nucléation puis mis en croissance en présence d'un peptisant de croissance, dans laquelle soit le peptisant de nucléation, soit le peptisant de croissance, soit les deux, est (sont) un matériau semblable au collagène recombinant sensiblement pur, exempt de structure hélicoïdale, et ledit peptisant ayant une séquence d'acides aminés comprenant plus de 4 acides aminés différents, dans laquelle ledit peptisant comprend la séquence d'acides aminés suivante :

GPP GEP GNP GSP GNQ GQP GNK GSP GNP GQP GNE GQP GQP GQN GQP
GEP GSN GPQ GSQ GNP GKN GQP GSP GSQ GSP GNQ GSP GQP GNP GQP
GEQ GKP GNQ GPA GG.

**2.** Emulsion d'halogénure d'argent tabulaire selon la revendication 1, dans laquelle ledit peptisant comprend la séquence d'acides aminés suivante :

GPPGEPGNPGSPGNQGQPGNKGSPGNPGQPGNEGQPGQPGQNGQPGEPGS
NGPQGSQGNPGKNGQPGSPGSQGSPGNQGSPGQPGNPGQPGEQGKPGNQG
PAGEPGNPGSPGNQGQPGNKGSPGNPGQPGNEGQPGQPGQNGQPGEPGSN
GPQGSQGNPGKNGQPGSPGSQGSPGNQGSPGQPGNPGQPGEQGKPGNQGP
AGEPGNPGSPGNQGQPGNKGSPGNPGQPGNEGQPGQPGQNGQPGEPGSNG
PQGSQGNPGKNGQPGSPGSQGSPGNQGSPGQPGNPGQPGEQGKPGNQGPA
GEPGNPGSPGNQGQPGNKGSPGNPGQPGNEGQPGQPGQNGQPGEPGSNGP
QGSQGNPGKNGQPGSPGSQGSPGNQGSPGQPGNPGQPGEQGKPGNQGPAG
G.

**3.** Protéine comprenant la séquence d'acides aminés suivante :

GPP GEP GNP GSP GNQ GQP GNK GSP GNP GQP GNE GQP GQP GQN GQP
GEP GSN GPQ GSQ GNP GKN GQP GSP GSQ GSP GNQ GSP GQP GNP GQP
GEQ GKP GNQ GPA GG.

**4.** Protéine comprenant la séquence d'acides aminés suivante :

GPPGEPGNPGSPGNQGQPGNKGSPGNPGQPGNEGQPGQPGQNGQPGEPGS
NGPQGSQGNPGKNGQPGSPGSQGSPGNQGSPGQPGNPGQPGEQGKPGNQG
PAGEPGNPGSPGNQGQPGNKGSPGNPGQPGNEGQPGQPGQNGQPGEPGSN
GPQGSQGNPGKNGQPGSPGSQGSPGNQGSPGQPGNPGQPGEQGKPGNQGP
AGEPGNPGSPGNQGQPGNKGSPGNPGQPGNEGQPGQPGQNGQPGEPGSNG
PQGSQGNPGKNGQPGSPGSQGSPGNQGSPGQPGNPGQPGEQGKPGNQGPA
GEPGNPGSPGNQGQPGNKGSPGNPGQPGNEGQPGQPGQNGQPGEPGSNGP
QGSQGNPGKNGQPGSPGSQGSPGNQGSPGQPGNPGQPGEQGKPGNQGPAG
G.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0610796 A
- US 5580712 A
- EP 0926543 A1
- EP 0926543 A

**Non-patent literature cited in the description**

- **CAPELLO, J. ; FERRARI, F.** Plastics from microbes. Hanser, 1994, 35-92
- **STRAUSBERG, R. L. ; INK, R. P.** *TIBTECH,* 1990, vol. 8, 53-57
- **MANIATIS T. ; FRITSCH, E. F. ; SAMBROOK, J.** Molecular cloning: A laboratory manual. Cold Spring Harbor Laboratory, 1982
- **GLUMOFF, V. ; MÄKELÄ, J. K. ; VUORIO, E.** Cloning of cDNA for rat proα1(III) collagen mRNA. Increased expression of type III collagen gene during induction of experimental granulation tissue. *Biochim Biophys Acta,* 1994, vol. 1217, 41-48
- **SANGER, F. ; NICKLEN, S. ; COULSON, A. R.** *Proc. Natl. Acad. Sci. USA,* 1977, vol. 74, 5463-5467
- **BECKER, D. M. ; GUARENTE, L.** High efficiency transformation of yeast by electoporation. *Methods in Enzymology,* 1991, vol. 194, 182-187
- **LEE, F. J. S.** *Biotechniques,* 1992, vol. 12 (5), 677
- **LAEMMLI, U. K.** Cleavage of structural proteins during the assembly of the head of bacteriophage T4. *Nature,* 1970, vol. 227, 680-685
- **SCHMITT, M. E. ; BROWN, T. A. ; TRUMPOWER, B. L.** A rapid and simple method for preparation of RNA from Saccharomyces cerevisiae. *Nucleic Acids Res.,* 1990, vol. 18 (10), 3091
- **SCORER, C. A. ; CLARE, J. J. ; MCCOMBIE, W. R ; ROMANOS, M. A. ; SREEKRISHNA, K.** Rapid Selection using G418 of high copy number transformants of Pichia pastoris for high-level foreign gene expression. *Bio/Technology,* 1994, vol. 12, 181-184
- **BUTKOWSKY R. J. ; NOELKEN, M. E. ; UDSON, B. G.** Estimation of the size of colagenous proteins by electrophoresis and gel chromatography. *Meth. Enzymol.,* 1982, vol. 82, 410-423
- **DE WOLF, F. A. ; KELLER R. C. A.** Characterization of the helical structures in gelatin networks and model polypeptides by circular dichroism. *Progr. Colloid Polym. Sci.,* 1996, vol. 102, 9-14

SEQUENCE LISTING

<110> FUJIFILM Manufacturing Europe B.V.

<120> Silver halide emulsions containing recombinant gelatin-like proteins

<130> P043276EP

<140> 99204382.8
<141> 1999-12-17

<150> US 09/219,849
<151> 1998-12-23

<160> 10

<170> PatentIn version 3.3

<210> 1
<211> 26
<212> DNA
<213> Artificial

<220>
<223> primer HLP-PA-FW

<400> 1
gcgctcgaga aaagagaggc tgaagc          26

<210> 2
<211> 108
<212> DNA
<213> Artificial

<220>
<223> primer OVL-PA-FW

<400> 2

gcgctcgaga aaagagaggc tgaagctggt ccacccggtg agccaggtaa cccaggatct          60

cctggtaacc aaggacagcc cggtaacaag ggttctccag gtaatcca          108

<210> 3

<211> 110
<212> DNA
<213> Artificial

<220>
<223> primer OVL-PA-RV

<400> 3

tgagaacctt gtggaccgtt ggaacctggc tcaccaggtt gtccgttctg accaggttga    60

ccaggttgac cttcgtttcc tggttgacct ggattacctg gagaaccctt    110


<210> 4
<211> 24
<212> DNA
<213> Artificial

<220>
<223> primer HLP-PA-RV

<400> 4
tgagaacctt gtggaccgtt ggaa    24

<210> 5
<211> 24
<212> DNA
<213> Artificial

<220>
<223> primer HLP-PB-FW

<400> 5
ttccaacggt ccacaaggtt ctca    24

<210> 6
<211> 115
<212> DNA
<213> Artificial

<220>
<223> primer OVL-PB-FW

<400> 6

ttccaacggt ccacaaggtt ctcagggtaa ccctggaaag aatggtcaac ctggatcccc    60

aggttcacaa ggctctccag gtaaccaagg ttcccctggt cagccaggta accct    115


<210> 7
<211> 108
<212> DNA
<213> Artificial

<220>
<223> primer OVL-PB-RV

<400> 7


gcgtctgcag tacgaattct attagccacc ggctggaccc tggtttcctg gtttaccttg          60

ttcacctggt tgaccagggt tacctggctg accaggggaa ccttggtt          108


<210> 8
<211> 26
<212> DNA
<213> Artificial

<220>
<223> primer HLP-PB-RV

<400> 8
gcgtctgcag tacgaattct attagc          26

<210> 9
<211> 104
<212> PRT
<213> Artificial

<220>
<223> monomer (P)

<400> 9


Gly Pro Pro Gly Glu Pro Gly Asn Pro Gly Ser Pro Gly Asn Gln Gly
1               5                   10                  15


Gln Pro Gly Asn Lys Gly Ser Pro Gly Asn Pro Gly Gln Pro Gly Asn
            20                  25                  30


Glu Gly Gln Pro Gly Gln Pro Gly Gln Asn Gly Gln Pro Gly Glu Pro
            35                  40                  45


Gly Ser Asn Gly Pro Gln Gly Ser Gln Gly Asn Pro Gly Lys Asn Gly
            50                  55                  60


Gln Pro Gly Ser Pro Gly Ser Gln Gly Ser Pro Gly Asn Gln Gly Ser
65                  70                  75                  80


Pro Gly Gln Pro Gly Asn Pro Gly Gln Pro Gly Glu Gln Gly Lys Pro
                    85                  90                  95


Gly Asn Gln Gly Pro Ala Gly Gly
                100


<210> 10
<211> 401
<212> PRT
<213> Artificial

<220>
<223> tetramer (P4)

<400> 10

Gly Pro Pro Gly Glu Pro Gly Asn Pro Gly Ser Pro Gly Asn Gln Gly
1               5                       10                  15

Gln Pro Gly Asn Lys Gly Ser Pro Gly Asn Pro Gly Gln Pro Gly Asn
                20                  25                  30

Glu Gly Gln Pro Gly Gln Pro Gly Gln Asn Gly Gln Pro Gly Glu Pro
            35                  40                  45

Gly Ser Asn Gly Pro Gln Gly Ser Gln Gly Asn Pro Gly Lys Asn Gly
        50                  55                  60

Gln Pro Gly Ser Pro Gly Ser Gln Gly Ser Pro Gly Asn Gln Gly Ser
65                  70                  75                  80

Pro Gly Gln Pro Gly Asn Pro Gly Gln Pro Gly Glu Gln Gly Lys Pro
                85                  90                  95

Gly Asn Gln Gly Pro Ala Gly Glu Pro Gly Asn Pro Gly Ser Pro Gly
            100                 105                 110

Asn Gln Gly Gln Pro Gly Asn Lys Gly Ser Pro Gly Asn Pro Gly Gln
            115                 120                 125

Pro Gly Asn Glu Gly Gln Pro Gly Gln Pro Gly Gln Asn Gly Gln Pro

130                135                140

Gly Glu Pro Gly Ser Asn Gly Pro Gln Gly Ser Gln Gly Asn Pro Gly
145             150             155             160

Lys Asn Gly Gln Pro Gly Ser Pro Gly Ser Gln Gly Ser Pro Gly Asn
            165             170             175

Gln Gly Ser Pro Gly Gln Pro Gly Asn Pro Gly Gln Pro Gly Glu Gln
            180             185             190

Gly Lys Pro Gly Asn Gln Gly Pro Ala Gly Glu Pro Gly Asn Pro Gly
            195             200             205

Ser Pro Gly Asn Gln Gly Gln Pro Gly Asn Lys Gly Ser Pro Gly Asn
    210             215             220

Pro Gly Gln Pro Gly Asn Glu Gly Gln Pro Gly Gln Pro Gly Gln Asn
225             230             235             240

Gly Gln Pro Gly Glu Pro Gly Ser Asn Gly Pro Gln Gly Ser Gln Gly
            245             250             255

Asn Pro Gly Lys Asn Gly Gln Pro Gly Ser Pro Gly Ser Gln Gly Ser
            260             265             270

Pro Gly Asn Gln Gly Ser Pro Gly Gln Pro Gly Asn Pro Gly Gln Pro
            275             280             285

Gly Glu Gln Gly Lys Pro Gly Asn Gln Gly Pro Ala Gly Glu Pro Gly
    290             295             300

Asn Pro Gly Ser Pro Gly Asn Gln Gly Gln Pro Gly Asn Lys Gly Ser
305             310             315             320

Pro Gly Asn Pro Gly Gln Pro Gly Asn Glu Gly Gln Pro Gly Gln Pro
            325             330             335

Gly Gln Asn Gly Gln Pro Gly Glu Pro Gly Ser Asn Gly Pro Gln Gly
            340             345             350

Ser Gln Gly Asn Pro Gly Lys Asn Gly Gln Pro Gly Ser Pro Gly Ser
            355             360             365

Gln Gly Ser Pro Gly Asn Gln Gly Ser Pro Gly Gln Pro Gly Asn Pro
            370             375             380

Gly Gln Pro Gly Glu Gln Gly Lys Pro Gly Asn Gln Gly Pro Ala Gly
385             390             395             400

Gly

.   .